Europäisches Patentamt
European Patent Office
Office européen des brevets

(19)

(11) Veröffentlichungsnummer: **0 235 116**
**A2**

(12)

# EUROPÄISCHE PATENTANMELDUNG

(21) Anmeldenummer: **87890009.1**

(22) Anmeldetag: **15.01.87**

(51) Int. Cl.[4]: **C 07 K 15/00**
C 07 K 15/14, A 61 K 39/395

(30) Priorität: **17.01.86 AT 108/86**

(43) Veröffentlichungstag der Anmeldung:
**02.09.87 Patentblatt 87/36**

(84) Benannte Vertragsstaaten:
**AT CH DE FR GB IT LI**

(71) Anmelder: **EBEWE Arzneimittel Gesellschaft mbH**
**A-4866 Unterach (AT)**

(72) Erfinder: **Hillebrand, Friedrich, Dr.**
**A-4866 Unterach am Attersee 214 (AT)**

**Mischak, Harald, Dipl.-Ing.**
**Andreas-Gruberstrasse 4**
**A-3100 St. Pölten (AT)**

(74) Vertreter: **Mrazek, Engelbert et al**
**Patentanwälte Dipl.-Ing. Dr. techn. Alfred Schütz, Dr. phil. Engelbert Mrazek, Dipl.-Ing. Walter Holzer, Dipl.-Ing. Otto Pfeifer**
**Fleischmanngasse 9 A-1040 Wien (AT)**

(54) **Desaktivierte Proteinderivate.**

(57) Neue, desaktivierte Proteinderivate der allgemeinen Formel

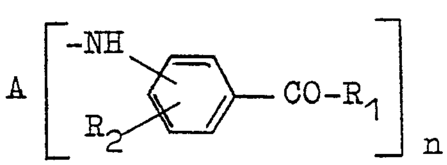

(I) ,

worin n eine Zahl von 1 bis 3 ist, A für den Rest eines Proteins vom Typ der Lectine oder Bakterientoxine steht, $R_1$ einen Rest eines Kohlenhydrats aus der Mono-, Di-, Tri-und Tetrasaccharide und deren Derivate umfassenden Gruppe bedeutet und $R_2$ ein Wasserstoffatom, ein $C_1$-$C_{10}$-Alkylrest oder die Nitrogruppe ist, mit denen eine Bindung der Lectine bzw. Bakterientoxine an der Zelloberfläche unterbunden wird; ferner zur Desaktivierung von Proteinen geeignete Verbindungen und Verfahren zur Herstellung dieser Proteinderivate und Verbindungen sowie Verwendung der desaktivierten Proteinderivate bei der Herstellung von Immunotoxinen.

EP 0 235 116 A2

Bundesdruckerei Berlin

**Beschreibung**

Desaktivierte Proteinderivate

Technisches Gebiet

Immunotoxine sind Verbindungen, die im allgemeinen aus einem immunologisch wirksamen Teil, wie z.B. einem Antikörper, vorzugsweise einem monoklonalen Antikörper, und einem Toxin bestehen. Immunotoxine werden zur selektiven Abtötung von neoplastischen Zellen im Rahmen der Krebstherapie eingesetzt.

Im Falle von Immunotoxinen, die aus einem Antikörper und einem unspezifischen Toxin bestehen, kann das Toxin gegenüber Zellen dadurch wirksam werden, daß a) das Immunotoxin über den Toxinanteil unspezifisch an Zellen gebunden und internalisiert wird oder

b) das Immunotoxin einer enzymatischen Lyse unterliegt,

wobei die Bindung zwischen Antikörper und unspezifischem Toxin gelöst und das Toxin freigesetzt wird; das Toxin wird auch in diesem Falle unspezifisch wirksam. In beiden Fällen tritt durch den Toxinanteil des eingesetzten Immunotoxins der Tod von Zellen ein, ohne daß eine selektive Steuerung des Toxins auf Zielzellen möglich ist.

Ziel der Erfindung

Die vorliegende Erfindung zielt darauf ab, neue desaktivierte Proteinderivate zu schaffen, die es bei einer bestimmten Gruppe von an sich unspezifisch zelltoxisch wirksamen Toxinen, nämlich bei Lectinen und Bakterientoxinen, ermöglichen, eine Bindung dieser Toxine an der Zelloberfläche zu unterbinden. Die Erfindung zielt weiterhin auf die Schaffung von zur Desaktivierung von Proteinen geeigneten Verbindungen und Verfahren zur Herstellung dieser Proteinderivate und Verbindungen sowie auf die Verwendung der desaktivierten Proteinderivate bei der Herstellung von Immunotoxinen ab.

Ausführliche Beschreibung der Erfindung

Unter Lectinen ist im Rahmen der Erfindung eine Gruppe von Proteinen zu verstehen, die mindestens zwei Bindungsstellen für Kohlenhydratgruppen aufweisen. Bakterientoxine sind von pathogenen Bakterien produzierte Proteine, die aus zwei Komponenten bestehen, von denen die eine Komponente toxisch wirkt und die andere Komponente, die selbst nicht toxisch ist, die Bindung des Toxins an die Zellmembran über Bindungsstellen für Kohlenhydrate sowie dessen Eindringen in die Zelle ermöglicht.

Den beiden Typen der vorgenannten Proteine, nämlich den Lectinen und den Bakterientoxinen, ist gemeinsam, daß sie zytotoxische Substanzen sind, die in ihren Strukturen zwei verschiedene Abschnitte aufweisen. Mit einem Strukturabschnitt können sie über Kohlenhydratbindungsstellen an die Zelloberfläche gebunden werden, wodurch eine Internalisation in die Zelle ermöglicht wird. Der andere Strukturabschnitt kann bestimmte, für die Zelle lebensnotwendige Enzymsysteme blockieren und dadurch in einer monomolekularen Reaktion den Zelltod herbeiführen. Durch die vorliegende Erfindung gelingt es, Kohlenhydratbindungsstellen in Proteinen vom Typ der Lectine und Bakterientoxine zu blockieren und dadurch dem Toxin die Fähigkeit zu nehmen, an die Zelloberfläche gebunden zu werden und in die Zelle einzudringen.

Die neuen, desaktivierten Proteinderivate gemäß der vorliegenden Erfindung haben die allgemeine Formel

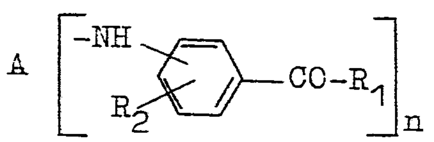

(I),

worin n eine Zahl von 1 bis 3 ist, A für den Rest eines Proteins vom Typ der Lectine oder Bakterientoxine steht, $R_1$ einen Rest eines Kohlenhydrats aus der Mono-, Di-, Tri- und Tetrasaccharide und deren Derivate umfassenden Gruppe bedeutet und $R_2$ ein Wasserstoffatom, ein $C_1$-$C_{10}$-Alkylrest oder die Nitrogruppe ist. In der allgemeinen Formel (I) kann A für den Rest eines beliebigen Proteins vom Typ der Lectine oder Bakterientoxine stehen. Bevorzugte Reste vom Typ der Lectine sind der Ricin-, der Abrin- und der Viscum Album Lectin-Rest, bevorzugte Reste vom Typ der Bakterientoxine sind Diphtherietoxin-, E.colitoxin-, Protein A- und Pseudomonastoxin-Reste.

Die neuen Proteinderivate der allgemeinen Formel (I) enthalten Proteine, deren Kohlenhydratbindungsstellen durch den Rest eines Aminobenzoesäurederivats, welches einen Rest $R_1$ enthält, blockiert sind. Bevorzugte Reste $R_1$ sind Galactose-, Lactose-, Mannose-, Galactosamin-, N-Acetylgalactosamin-, Glucosamin- oder N-Acetylglucosamin-Reste. Bevorzugte Alkylreste $R_2$ haben 1 bis 5 Kohlenstoffatome, insbesondere 1, 2 oder 3 Kohlenstoffatome, wie Methyl, Ethyl und Propyl.

In den erfindungsgemäßen desaktivierten Proteinderivaten (I) ist der auf das Zellsystem wirkende Strukturanteil des Proteinrestes A zwar intakt, kann aber infolge des Vorliegens der Blockierungsgruppe mit dem Rest $R_1$ nicht in die Zelle eindringen und daher dort nicht wirksam werden.

Die neuen Proteinderivate der allgemeinen Formel (I) sind für die Verwendung bei der Herstellung von

Immunotoxinen wertvoll.

Um die Toxine der erfindungsgemäßen desaktivierten Proteinderivate der allgemeinen Formel (I), die einen Rest A eines Proteins vom Typ der Lectine oder Bakterientoxine enthalten, zellwirksam werden zu lassen, benötigen diese blockierten Toxine einen spezifischen Carrier, der das Eindringen in die Zelle ermöglicht. Das Fehlen eines solchen Carriers schließt eine unspezifische, toxische Wirkung des Toxinanteils der neuen desaktivierten Proteinderivate aus. Beispiele für solche Carrier, die eine zellspezifische, toxische Wirkung der erfindungsgemäßen desaktivierten Proteinderivate der allgemeinen Formel (I) auslösen, sind monoklonale Antikörper.

Zur Herstellung der neuen desaktivierten Proteinderivate der allgemeinen Formel (I) setzt man ein Azidobenzoesäurederivat der allgemeinen Formel

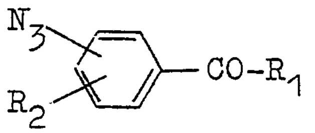 (II) ,

worin $R_1$ und $R_2$ die oben angegebenen Bedeutungen haben, mit einem Protein vom Typ der Lectine oder Bakterientoxine zu einem Proteinderivat der allgemeinen Formel (I) um. Die bei diesem Herstellungsverfahren verwendeten Proteine (Toxine) sind im allgemeinen käuflich zu erwerben, können aber auch durch Extraktion aus Pflanzen bzw. deren Samen sowie auch aus Bakterien gewonnen werden. Die Reinigung erfolgt zweckmäßig affinitätschromatographisch, vorzugsweise über eine Säule, die mit dem entsprechenden immobilisierten Kohlenhydrat gefüllt ist, und mittels Ionenaustauschchromatographie, vorzugsweise unter Verwendung von CM-Sepharose.

Die beim obigen Herstellungsverfahren eingesetzten Azidobenzoesäurederivate der allgemeinen Formel (II) sind neu und sind gleichfalls ein Gegenstand der vorliegenden Erfindung. Sie können hergestellt werden, indem man Azidobenzoesäuren der Formel

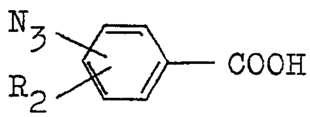 (III),

oder deren reaktive Derivate, worin $R_2$ die oben angegebene Bedeutung hat, mit Kohlenhydraten aus der Mono-, Di-, Tri-und Tetrasaccharide und deren Derivate umfassenden Gruppe umsetzt. Bevorzugte reaktive Derivate von Azidobenzoesäuren der Formel (III) sind N-Hydroxysuccinimidester der entsprechenden Azidobenzoesäure.

Nach einer besonderen Ausführungsform des erfindungsgemäßen Verfahrens zur Herstellung der Azidobenzoesäurederivate der allgemeinen Formel (II) erfolgt ein Umsetzen der N-Hydroxysuccinimidester mit Aminozuckern in $H_2O$/DMF-Lösungsmittelgemischen.

Die Reinigung der Verbindungen der allgemeinen Formel (II) erfolgt zweckmäßig durch Säulenchromatographie auf Kieselgelderivaten mit einem Gemisch aus Wasser und Acetonitril als Eluens.

Die Umsetzung der Azidobenzoesäurederivate der allgemeinen Formel (II) mit den einen Rest A enthaltenden Proteinen (Toxinen) kann durch Lichteinwirkung beschleunigt werden. Zweckmäßig arbeitet man bei Verwendung von Azidobenzoesäurederivaten der allgemeinen Formel (II), worin $R_2$ ein Wasserstoffatom oder einen $C_1$-$C_{10}$-Alkylrest bedeutet, unter Bestrahlung mit UV-Licht in homogener, wässeriger Lösung. Bei Verwendung von Azidobenzoesäurederivaten der allgemeinen Formel (II), worin $R_2$ die Nitrogruppe bedeutet, ist ein Arbeiten unter Bestrahlung mit sichtbarem Licht in homogener, wässeriger Lösung vorteilhaft. Die Dauer der Bestrahlung zur Photoaktivierung der Azidobenzoesäurederivate der allgemeinen Formel (II) kann bei 4° C mehrere Stunden betragen.

Die Abtrennung und Reinigung der neuen desaktivierten Proteinderivate der allgemeinen Formel (I) erfolgt zweckmäßig durch Affinitätschromatographie, am besten über eine Säule gefüllt mit dem entsprechenden immobilisierten Kohlenhydrat.

Beispiele

1) Herstellung von N-4-Azidobenzoylgalactosamin

100 mg Galactosaminhydrochlorid werden in 5 ml $H_2O$ dest. gelöst und 150 mg N-Hydroxisuccinimidester der 4-Azidobenzoesäure sowie 100 mg Dicyclohexylcarbodiimid werden in 30 ml Dimethylformamid gelöst. Die beiden Lösungen werden in einem geeigneten Gefäß vereint und der pH-Wert dieser Lösung mittels $NaHCO_3$ auf 7 - 7,5 gestellt.

Diese Lösung wird im verschlossenen Gefäß bei Raumtemperatur mindestens 12 h gerührt. Nach dem Ende der Reaktion werden die Lösungsmittel im Wasserstrahlvakuum entfernt und der Rückstand in 50 ml $H_2O$ dest. aufgenommen.

Nach dem Abtrennen der unlöslichen Bestandteile wird die so gewonnene Lösung auf eine 10 x 1 cm Säule, gefüllt mit LiChroprep® RP-18 (Korngröße 0,04 - 0,o63 mm) und mit $H_2O$ dest. equillibriert, aufgegeben und nach Erreichen einer stabilen Grundlinie mittels Acetonitril-Gradienten (0 - 100 % Acetonitril linear über 500 ml) eluiert. Die so gewonnene reine Substanz wird lyophilisiert und bei 4 °C aufbewahrt (Ausbeute 114 mg, d.i. 84 % d. Th.). N-4-Azidobenzoylgalactosamin wurde durch IR-, NMR- und UV-Spektroskopie sowie durch eine CHN-Analyse charakterisiert:

IR-Spektrum: Azidbande bei 2120 $cm^{-1}$

UV-Spektrum: Maximum bei 270 nm

NMR-Spektrum: Protonen des parasubstituierten Aromaten bei 7,05-8,14 ppm, Verhältnis der Integrale der aromatischen Protonen zu denen des Kohlenhydratanteils 4 zu 7.

| CHN-Analyse: | C | H | N |
|---|---|---|---|
| | % | % | % |
| berechnet: | 48,15 | 4,97 | 17,28 |
| gefunden: | 46,16 | 4,92 | 16,43 |

2) Herstellung von N-5-Azido-2-nitrobenzoylgalactosamin

100 mg Galactosaminhydrochlorid werden in 5 ml $H_2O$ dest. gelöst und 175 mg n-Hydroxisuccinimidester der 5-Azido-2-nitrobenzoesäure sowie 100 mg Dicyclohexylcarbodiimid werden in 30 ml Dioxan gelöst. Die beiden Lösungen werden in einem geeigneten Gefäß vereint und der pH-Wert der Lösung mittels $NaHCO_3$ auf 7 - 7,5 gestellt.

Diese Lösung wird im verschlossenen Gefäß bei Raumtemperatur mindestens 12 h gerührt. Nach dem Ende der Reaktion werden die Lösungsmittel im Wasserstrahlvakuum entfernt und der Rückstand in 50 ml $H_2O$ dest. aufgenommen.

Nach dem Abtrennen der unlöslichen Bestandteile wird die so gewonnene Lösung auf eine 10 x 1 cm Säule, gefüllt mit LiChroprep®RP-18 (Korngröße 0,04 - 0,063 mm) und mit $H_2O$ dest. equillibriert, aufgegeben und nach Erreichen einer stabilen Grundlinie mittels Acetonitril-Gradienten (0 - 100 % Acetonitril linear über 1000 ml) eluiert. Die so gewonnene, reine Substanz wird lyophilisiert und bei 4 °C aufbewahrt (Ausbeute 122 mg, d.i. 78% d.Th.) N-5-Azido-2-nitrobenzoylgalactosamin wurde durch IR-, NMR- und UV-Spektroskopie sowie durch eine CHN-Analyse charakterisiert:

IR-Spektrum: Azidbande bei 2120 $cm^{-1}$ sowie $NO_2$-Bande bei 1340 $cm^{-1}$

UV-Spektrum: Maximum bei 315 nm

NMR-Spektrum: Protonen des meta-para-disubstituierten Aromaten bei 6,98-7,88 ppm, Verhältnis der Integrale der aromatischen Protonen zu denen des Kohlenhydratanteils 3 zu 7.

| CHN-Analyse: | C | H | N |
|---|---|---|---|
| | % | % | % |
| berechnet: | 42,28 | 4,09 | 18,97 |
| gefunden: | 42,14 | 4,02 | 20,12 |

3) Herstellung von an der Galactosebindungsstelle blockiertem Ricin

50 mg Ricin, gelöst in 1 ml 10 mM Tris/HCl-Puffer pH 7,7, werden in einem geeigneten Gefäß mit 3 mg N-4-Azidobenzoylgalactosamin versetzt und mit einer 40-W Germizidleuchtstoffröhre$_{254}$ ohne Filterabdekkung aus 5 cm Entfernung mindestens 30 min. belichtet.

Nach Ende der Reaktion wird überschüssiges zerstörtes N-4-Azidobenzoylgalactosamin durch Entsalzen über eine 10 x 2 cm Säule gefüllt mit Biogel P6DG entfernt und das entstandene Produkt mittels Affinitätschromatographie über eine 5 x 3 cm Säule gefüllt mit Lactose-Sepharose gereinigt.

Das so gewonnene Produkt wird in einer Konzentration von 1 mg/ml in 50 mM Natriumphosphatpuffer pH 7,2 unter Zusatz von 0,02 % $NaN_3$ bei 4° C aufbewahrt. Ausbeute: 3,7 mg d.i. 7,4 % d.Th.

An der Kohlenhydratbindungsstelle blockiertes Ricin wurde durch seine Aktivität im zellfreien Proteinsynthesesystem (Reticulozytenlysat aus Kaninchen), seine verminderte Toxizität auf Zellinien (Human Zellinie HL 60) und sein Molekulargewicht in der SDS-Elektrophorese sowie durch seine, bei der Reinigung ausgenützte, Unfähigkeit entsprechende Kohlenhydrate zu binden charakterisiert.

| | Ricin | "blockiertes" Ricin |
|---|---|---|
| 50 %-Hemmung der Proteinsynthese im zellfreien System | $10^{-10}M$ | $10^{-10}M$ |
| $LD_{50}$ auf Zellinien HL 60 | $4.10^{-12}M$ | $> 10^{-8}M$ |
| Molekulargewicht (SDS-Elektrophorese) | 60 und 62 kD* | 60 und 62 kD |

* Von einer der zwei Ricin-Untereinheiten gibt es zwei, nur im Molekulargewicht unterschiedliche Formen, eine Doppelbande bei 60 und 62 kD ist daher typisch für Ricin und keinesfalls als Verunreinigung anzusehen.

4) Herstellung von an der Galactosebindungsstelle blockiertem Ricin mittels N-5-Azido-2-nitrobenzoylgalactosamin

50 mg Ricin werden in 1 ml 10 mM Tris/HCl-Puffer pH 7,7 gelöst, mit 3,5 mg N-5-Azido-2-nitrobenzoylgalactosamin versetzt und in einem geeigneten Gefäß aus 20 cm Entfernung mit einer 500 W Quecksilberdampflampe mit Emissionsmaximum bei 320 nm unter Verwendung einer Fokussierlinse mindestens 20 min. belichtet.

Nach Ende der Reaktion wird überschüssiges zerstörtes N-5-Azido-2-Nitrobenzoylgalactosamin durch Entsalzen über eine 10 x 2 cm Säule gefüllt mit Biogel P6DG entfernt und das entstandene Produkt mittels Affinitätschromatographie über eine 5 x 3 cm Säule gefüllt mit Lactose-Sepharose gereinigt.

Das so gewonnene Produkt wird in einer Konzentration von 1 mg/ml in 50 mM Natriumphosphatpuffer pH 7,2 unter Zusatz von 0,02 % $NaN_3$ bei 4 °C aufbewahrt. Ausbeute: 2,9 mg, d.i. 5,8 % d.Th.

An der Kohlenhydratbindungsstelle blockiertes Ricin wurde durch seine Aktivität im zellfreien Proteinsynthesesystem (Reticulozytenlysat aus Kaninchen), seine verminderte Toxizität auf Zellinien (Human Zellinie HL 60) und sein Molekulargewicht in der SDS-Elektrophorese sowie durch seine, bei der Reinigung ausgenützte, Unfähigkeit entsprechende Kohlenhydrate zu binden charakterisiert.

| | Ricin | "blockiertes" Ricin |
|---|---|---|
| 50 %-Hemmung der Proteinsynthese im zellfreien System | $10^{-10}M$ | $10^{-10}M$ |
| $LD_{50}$ auf Zellinien HL 60 | $4.10^{-12}M$ | $> 10^{-8}M$ |
| Molekulargewicht (SDS-Elektrophorese) | 60 und 62 kD* | 60 und 62 kD |

* Von einer der zwei Ricin-Untereinheiten gibt es zwei, nur im Molekulargewicht unterschiedliche Formen, eine Doppelbande bei 60 und 62 kD ist daher typisch für Ricin und keinesfalle als Verunreinigung anzusehen.

5) Herstellung von an der Kohlenhydratbindungsstelle blockiertem Abrin mittels N-5-Azido-2-nitrobenzoylgalactosamin

20 mg Abrin werden in 1 ml 20 mM Natriumphosphatpuffer (pH 6,5) suspendiert und in einem entsprechendem Gefäß mit 2 mg N-5-Azido-2-nitrobenzoylgalactosamin versetzt.

Die Photoaktivierung erfolgt mit einer 500 W Quecksilberhochdrucklampe aus etwa 20 cm Entfernung unter intensiver Kühlung auf 10°C.

Das überschüssige Reagens wird nach vollständiger Photolyse durch Dialyse gegen den o. a. Puffer entfernt. Das blockierte wird vom noch intakten Abrin über eine 1 x 5 cm Säule, gefüllt mit an epoxi-aktivierte Sepharose gekoppelter Lactose, getrennt, wobei dieser Vorgang drei mal wiederholt wird.

Dieses Produkt wird in 10 mM Natriumphosphatpuffer unter Zusatz von 0,8 % NaCl und 0,02 % $NaN_3$ bei 4°C aufbewahrt.

Das an der Kohlenhydratbindungsstelle blockierte Abrin wurde durch folgende Parameter charakterisiert:
- Molekulargewicht in der SDS-Elektrophorese
- Aktivität im zellfreien Proteinsynthesesystem

- Verminderte Toxizität auf der Zellinie HL 60
- Unfähigkeit, Lactose zu binden.

| | "blockiertes Abrin" | "Abrin" |
|---|---|---|
| Molekulargewicht (SDS-Elektrophorese) | 59 kD | 59 kD |
| $LD_{50}$ auf Zellinien (Linie HL 60) | $2.10^{-7}$M | $5.10^{-10}$M |
| 50 % Hemmung der Proteinbiosynthese | $3.10^{-11}$M | $3.10^{-11}$M |

6) Herstellung von an der Kohlenhydratbindungsstelle blockiertem Viscum Album Lectin

20 mg Viscum Album Lectin werden in 1 ml 20 mM Natriumphosphatpuffer (pH 6,5) suspendiert und in einem entsprechenden Gefäß mit 2 mg N-5-Azido-2-Nitrobenzoylgalactosamin versetzt.

Die Photoaktivierung erfolgt mit einer 500 W Quecksilberhochdruckdampflampe aus etwa 20 cm Enfernung unter intensiver Kühlung auf 10°C.

Das überschüssige Reagens wird nach vollständiger Photolyse durch Dialyse gegen den o. a. Puffer entfernt. Das blockierte Viscum Album Lectin wird von noch intaktem Viscum Album Lectin über eine 1 x 5 cm Säule, getrennt, wobei dieser Vorgang drei mal wiederholt wird.

Dieses Produkt wird in 10 mM Natriumphosphatpuffer unter Zusatz von 0,8 % NaCl und 0,02 % $NaN_3$ bei 4°C aufbewahrt. Ausbeute: 1,4 mg, das sind 7 % d. Th.

Das an der Galactosebindungsstelle blockierte Viscum Album Lectin wurde durch folgende Parameter charakterisiert:
- Molekulargewicht in der SDS-Elektrophorese
- Aktivität im zellfreien Proteinsynthesesystem
- Verminderte Toxizität auf der Zellinie HL 60
- Unfähigkeit, Lactose zu binden.

| | "blockiertes" Viscum Album Lectin | Viscum Album Lectin |
|---|---|---|
| Molekualargewicht (SDS-Elektrophorese) | 60 kD | 60 kD |
| $LD_{50}$ auf Zellinien (Linie HL 60) | $7.10^{-8}$M | 1,1 $10^{-10}$M |
| 50 % Hemmung der Proteinbiosynthese | $3.10^{-10}$M | $10^{-10}$M |

**Patentansprüche**

1. Proteinderivate der allgemeinen Formel

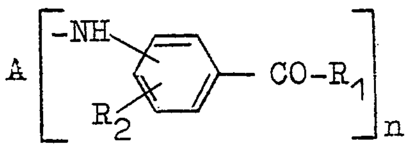

(I) ,

worin n eine Zahl von 1 bis 3 ist, A für den Rest eines Proteins vom Typ der Lectine oder Bakterientoxine steht, $R_1$ einen Rest eines Kohlenhydrats aus der Mono-, Di-, Tri-und Tetrasaccharide und deren Derivate umfassenden Gruppe bedeutet und $R_2$ ein Wasserstoffatom, ein $C_1$-$C_{10}$-Alkylrest oder die Nitrogruppe ist.

2. Proteinderivate nach Anspruch 1, worin n eine Zahl von 1 bis 3 ist, A für den Ricin- oder Abrinrest steht und $R_1$ und $R_2$ die oben angegebenen Bedeutungen haben.

3. Proteinderivate nach Anspruch 1, worin n eine Zahl von 1 bis 3 ist, A für den Diphtherietoxin-, E.colitoxin-, Protein A- oder Pseudomonastoxin-Rest steht und $R_1$ und $R_2$ die oben angegebenen Bedeutungen haben.

4. Proteinderivate nach einem der Ansprüche 1 bis 3, dadurch gekennzeichnet, daß $R_1$ einen Galactose, Lactose-, Mannose-, Galactosamin-, N-Acetylgalactosamin-, Glucosamin- oder N-Acetylglucosamin-Rest bedeutet, n eine Zahl von 1 bis 3 ist und A und $R_2$ die oben angegebenen Bedeutungen haben.

5. Azidobenzoesäurederivate der allgemeinen Formel

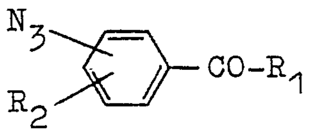

(II) ,

worin $R_1$ einen Rest eines Kohlenhydrats aus der Mono-, Di-, Tri- und Tetrasaccharide und deren Derivate umfassenden Gruppe bedeutet und $R_2$ ein Wasserstoffatom, ein $C_1$-$C_{10}$-Alkylrest oder die Nitrogruppe ist.

6. Azidobenzoesäurederivate nach Anspruch 5, worin $R_1$ einen Galactose-, Lactose-, Mannose-, Galactosamin-, N-Acetylgalactosamin-, Glucosamin- oder N-Acetylglucosamin-Rest bedeutet und $R_2$ die oben angegebene Bedeutung hat.

7. Verfahren zur Herstellung von neuen Proteinderivaten der allgemeinen Formel

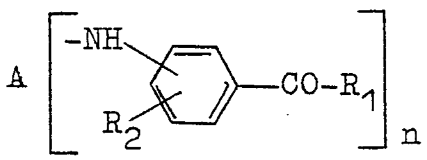

(I) ,

worin n eine Zahl von 1 bis 3 ist, A für den Rest eines Proteins vom Typ der Lectine oder Bakterientoxine steht, $R_1$ einen Rest eines Kohlenhydrats aus der Mono-, Di-, Tri- und Tetrasaccharide und deren Derivate umfassenden Gruppe bedeutet und $R_2$ ein Wasserstoffatom, ein $C_1$-$C_{10}$-Alkylrest oder die Nitrogruppe ist, dadurch gekennzeichnet, daß man ein Azidobenzoesäurederivat der allgemeinen Formel

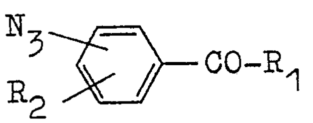

(II) ,

worin $R_1$ und $R_2$ die oben angegebenen Bedeutungen haben, mit einem Protein vom Typ der Lectine oder Bakterientoxine zu einem Proteinderivat der allgemeinen Formel (I) umsetzt.

8. Verfahren nach Anspruch 7, dadurch gekennzeichnet, daß man als Protein vom Typ der Lectine Ricin, Abrin oder Viscum Album Lectin einsetzt.

9. Verfahren nach Anspruch 7, dadurch gekennzeichnet, daß man als Protein vom Typ der Bakterientoxine Diphtherietoxin, E.colitoxin, Pseudomonastoxin oder Protein A einsetzt.

10. Verfahren nach einem der Ansprüche 7 bis 9, dadurch gekennzeichnet, daß man als Azidobenzoesäurederivat der allgemeinen Formel (II) ein solches einsetzt, worin $R_2$ ein Wasserstoffatom oder einen $C_1$-$C_{10}$-Alkylrest bedeutet und $R_1$ die oben angegebene Bedeutung hat, und die Umsetzung unter Bestrahlung mit UV-Licht in homogener, wässeriger Lösung durchführt.

11. Verfahren nach einem der Ansprüche 7 bis 9, dadurch gekennzeichnet, daß man als Azidobenzoesäurederivat der allgemeinen Formel (II) ein solches einsetzt, worin $R_2$ die Nitrogruppe bedeutet und $R_1$ die oben angegebene Bedeutung hat, und die Umsetzung unter Bestrahlung mit sichtbarem Licht in homogener, wässeriger Lösung durchführt.

12. Verfahren zur Herstellung von Azidobenzoesäurederivaten der allgemeinen Formel

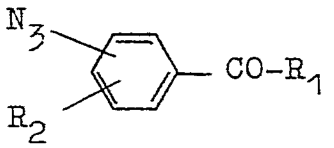

(II) ,

worin $R_1$ einen Rest eines Kohlehydrats aus der Mono-, Di-, Tri- und Tetrasaccharide und deren Derivate umfassenden Gruppe bedeutet und $R_2$ ein Wasserstoffatom, ein $C_1$-$C_{10}$-Alkylrest oder die Nitrogruppe ist, dadurch gekennzeichnet, daß man Azidobenzoesäuren der Formel

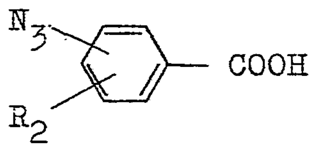

(III) ,

oder deren reaktive Derivate, worin $R_2$ ein Wasserstoffatom, ein $C_1$-$C_{10}$-Alkylrest oder die Nitrogruppe bedeutet, mit Kohlenhydraten aus der Mono-, Di-, Tri- und Tetrasaccharide und deren Derivate umfassenden Gruppe umsetzt.

13. Verfahren nach Anspruch 12, dadurch gekennzeichnet, daß man als reaktive Derivate von Azidobenzoesäuren der Formel (III) N-Hydroxysuccinimidester der entsprechenden Azidobenzoesäure einsetzt.

14. Verfahren nach Anspruch 12 oder 13, dadurch gekennzeichnet, daß man als Kohlenhydrate aus der Mono-, Di-, Tri-und Tetrasaccharide und deren Derivate umfassenden Gruppe Galactose, Lactose, Mannose, Galactosamin, N-Acetylgalactosamin, Glucosamin oder N-Acetylglucosamin oder deren Derivate einsetzt.

15. Verwendung von Proteinderivaten der allgemeinen Formel

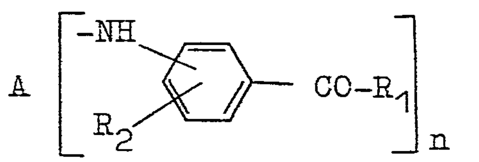

(I) ,

worin n eine Zahl von 1 bis 3 ist, A für den Rest eines Proteins vom Typ der Lectine oder Bakterientoxine steht, $R_1$ einen Rest eines Kohlenhydrats aus der Mono-, Di-, Tri-und Tetrasaccharide und deren Derivate umfassenden Gruppe bedeutet und $R_2$ ein Wasserstoffatom, ein $C_1$-$C_{10}$-Alkylrest oder die Nitrogruppe ist, bei der Herstellung von Immunotoxinen.